# EUROPÄISCHE PATENTSCHRIFT

(11) **EP 0 683 678 B1**
(45) Veröffentlichungstag und Bekanntmachung des Hinweises auf die Patenterteilung: **15.07.1998**
(21) Anmeldenummer: 94906906.6
(22) Anmeldetag: 05.02.1994
(51) Int. Cl.: A61L 2/04, A61L 2/18

(54) **VERFAHREN ZUR INAKTIVIERUNG VON VIREN, DIE NICHT MIT LIPIDHÜLLEN VERSEHEN SIND**
METHOD FOR INACTIVATING VIRUSES DEVOID OF LIPID ENVELOPES
PROCEDE POUR L'INACTIVATION DE VIRUS NE COMPORTANT PAS D'ENVELOPPES LIPIDIQUES

(30) Priorität: 09.02.1993 DE 4303609; 03.06.1993 DE 4318435
(43) Veröffentlichungstag der Anmeldung: 29.11.1995
(73) Patentinhaber: OCTAPHARMA AG, 8866 Ziegelbrücke (CH)
(72) Erfinder: STADLER, Monika, A-2435 Wienerherberg (AT); SCHWINN, Horst, D-35039 Marburg (DE); JOSIC, Djuro, A-1020 Wien (AT); GEHRINGER, Werner, A-1160 Wien (AT); BAL, Frederic, A-1080 Wien (AT)
(74) Vertreter: Meyers, Hans-Wilhelm, Dr.Dipl.-Chem.
(86) Internationale Anmeldenummer: EP9400328
(87) Internationale Veröffentlichungsnummer: WO9417834

(56) Entgegenhaltungen:
- EP-A- 0 131 740
- EP-A- 0 306 778
- EP-A- 0 367 840
- EP-A- 0 378 208
- EP-A- 0 479 597
- DATABASE WPI Section Ch, Week 9034, Derwent Publications Ltd., London, GB; Class B04, AN 90-257580 & JP,A,2 180 833 (GREEN CROSS) 13. Juli 1990
- Murphy et al 1993, J. Med. Virol. 41; 61-64

## Beschreibung

Gegenstand der vorliegenden Anmeldung ist ein Verfahren zur Inaktivierung von Viren, die nicht mit einer Lipidhülle versehen sind, in Protein enthaltenden Zusammenstellungen aus Blut, Blutplasma oder ähnlichen natürlichen Quellen.

Die EP 0 131 740 B1 beschreibt ein Verfahren zur Inaktivierung von Viren in labile Proteine enthaltenden Zusammensetzungen. Die zu inaktivierenden Viren enthalten Lipide und können insbesondere mit einer Lipidhülle versehen sein. Die von Lipid enthaltenden Viren zu befreiende Zusammensetzung stammt aus einer natürlichen Quelle, die ausgewählt ist aus der Gruppe Vollblut, Blutplasma, Plasmakonzenzentrat, Präzipitat irgendeiner Fraktionierung solchen Plasmas, Überstand aus irgendeiner Fraktionierung solchen Plasmas, Serum, Kryopräzipitat, Zell-Lysat, in Blutzellen induzierte Proteine, Produkt einer nicht Blut entstammenden normalen oder Krebszelle oder Produkt eines Gen-Splicing-Vorgangs. Das in der EP 0 131 740 B1 beschriebene Verfahren besteht aus einem Inkontaktbringen der labiles Protein enthaltenden Zusammensetzung mit einer wirksamen Menge eines Di- oder Trialkylphosphats für eine Zeitdauer, die ausreichend ist, um die labiles Protein enthaltende Zusammensetzung frei von Lipid enthaltenden Viren zu machen, ohne eine wesentliche Proteindenaturierung herbeizuführen. Dabei kann das beschriebene Verfahren zur Inaktivierung von Lipid enthaltenden Viren mit einer Wärmebehandlung bei 50 bis 70°C und einer Zeitdauer von mindestens 5 Stunden kombiniert werden.

Bei Präparationen der eingangs genannten Art ist es jedoch zunehmend von Bedeutung, auch solche Viren zu inaktivieren, die kein Lipid enthalten, insbesondere solche, die keine Lipidhülle aufweisen. Zur Gruppe der keine Lipidhülle aufweisenden Viren, die als nicht Lipid enthaltende Viren im Sinne der EP 0 131 740 B1 aufzufassen sind, zählen insbesondere Hepatitis-A-, Parvo- wie Parvorirus B 19, Polioviren. Diese Viren können als Krankheitserreger in Blut, Plasma, Serum, Kryopräzipitat, Zell-Lysat und ähnlichen natürlichen Quellen vorkommen.

Ein Ziel der vorliegenden Erfindung ist es, ein Verfahren bereitzustellen, das es erlaubt, Viren, die keine Lipidhülle oder nur wenige Lipide enthalten, in bestimmten Präparationen zu inaktivieren. Zu solchen Präparationen sollen insbesondere labile Proteine enthaltende Zusammensetzungen aus Vollblut, Blutplasma, Plasmakonzentrat, Präzipitat irgendeiner Fraktionierungsstufe solchen Plasmas, Überstand aus irgendeiner Fraktionierung solchen Plasmas, Serum, Kryopräzipitat, Zell-Lysat oder ähnlichen natürlichen Quellen gerechnet werden.

Überraschenderweise wird dieses Ziel durch ein Verfahren, das Viren, die nicht mit einer Lipidhülle versehen sind, in Protein enthaltenden Zusammenstellung aus Blut, Blutplasma oder ähnlichen natürlichen Quellen, erreicht, wobei eine Behandlung der Quelle mit einer wirksamen Menge von Di- oder Trialkylphosphaten und gegebenenfalls Benetzungsmitteln, gleichzeitig oder aufeinanderfolgend, bei einer erhöhten Temperatur im Bereich von 55°C bis 67°C für eine Zeitdauer von 5 bis 30 Stunden.

Die Mengen an Di- oder Trialkylphosphat beträgt vorzugsweise zwischen 0,001 % und 1 %. Zur Durchführung des Verfahrens werden vorzugsweise Temperaturen zwischen 60°C und 65°C eingestellt. Die Zeitdauer der Wärmebehandlung liegt vorzugsweise bei mindestens 10 Stunden.

Die Proteinfraktion, in der die keine Lipide enthaltenden Viren inaktiviert werden sollen, stammt insbesondere aus natürlichen Quellen der eingangs genannten Art und kann insbesondere durch Präzipitations- oder chromatographische Verfahren vor der Inaktivierungsreaktion mit dem entsprechenden Protein angereichert worden sein.

Insbesondere hat sich dabei eine Anreicherung der Proteinfraktion mit chromatographischen Methoden, wie sie in der EP 0 367 840 A1 beschrieben sind, bewährt. Dabei wird davon ausgegangen, daß die natürliche Quelle, die das Protein liefert, einer Anionenustauscherchromatographie unterworfen wird. Insbesondere haben sich mit Diethylaminoethylen-Gruppen modifizierte chromatographische Tägermaterialien bewährt.

Die natürliche Quelle oder die aus der natürlichen Quelle angereicherte Fraktion wird dann dem oben beschriebenen Virusinaktivierungsverfahren durch Wärmebehandlung und Behandlung mit Di- oder Trialkylphosphaten unterworfen.

Es hat sich als vorteilhaft erwiesen, die Behandlung mit Di- oder Trialkylphosphaten in Gegenwart von Benetzungsmitteln durchzuführen. Als Alkylphosphate kommen insbesondere die in der EP 0 131 740 B1 genannten Phosphate in Betracht wie Dialkylphosphate oder Trialkylphosphate mit Alkylgruppen, die 1 bis 10 Kohlenstoffatome enthalten, inbesondere 2 bis 10 Kohlenstoffatome. Insbesondere kommen Trialkylphosphate wie Tri-(n-butyl)phosphat, Tri-(t-butyl)phosphat, Tri-(n-hexylphosphat, Tri-(2-ethylhexyl)phosphat, Tri-(n-decyl)phosphat in Betracht. Auch gemische Trialkylphosphate sind geeignet. In ähnlicher Weise können auch die entsprechend substituierten Dialkylphosphate oder Mischungen entsprechender Di- oder Trialkylphosphate eingesetzt werden.

Als Benetzungsmittel kommen insbesondere nicht toxische Detergenzien in Betracht. Als nicht ionische Detergenzien, die in Mengen von mindestens 0,1 Gew.-% vorliegen sollten, sind die folgenden Derivate zu nennen: Polyoxyethylenderivate von Fettsäuren, Partialester von Sorbitolanhydrid, z. B. Produkte unter dem Handelsnamen Tween 80, Tween 20 und Polysorbat 80 bekannt sind sowie nicht inonische, Öl lösliche Benetzungsmittel, insbesondere solche, die unter dem Handelsnamen TritonX100 (ethoxylierte Alkylphenole) bekannt sind. Auch Zwitterreagenzien, beispielsweise Sulfobetaine wie n-Dodecyl-N, n-dimethyl-2-ammonio-1-ethansulfonat oder Derivate davon oder nicht ionische Detergenzien wie Octyl-β-D-glucopyranoside kommen in Betracht. Die Menge des Benetzungsmittels beträgt vorzugsweise 0,01 % bis 10 %.

Insbesondere bevorzugt sind Kombinationen von Tri(nbutyl)phosphat und Tween oder Natriumcholat/TNBP (Tri-(n-butyl)phosphat).

Es hat sich als vorteilhaft erwiesen, die Behandlung mit erhöhter Temperatur in Gegenwart von unterstützenden Substanzen wie Saccharose, Sorbitol oder kurzkettigen neutralen Aminosäuren durchzuführen. Grundsätzlich können die in der EP 0 018 561 und/oder DE 40 01 451 A1 genannten Stabilisierungsfaktoren in den angegebenen Mengenbereichen verwendet werden. Dabei kann die Konzentration der unterstützenden Substanzen (Stabilisierungsfaktoren) sehr hoch sein, vorzugsweise beträgt beispielsweise die Saccharosekonzentration bis zu 200 Gew.-%. Als kurzkettige Aminosäuren kommen insbesondere Glycin, Lysin und/oder Arginin in Frage.

Es hat sich überraschenderweise herausgestellt, daß die Behandlung der Protein enthaltenden Zusammenstellungen aus Blut, Blutplasma oder ähnlichen natürlichen Quellen bei einer erhöhten Temperatur auch ohne Zusatz von Calciumionen erfolgen kann. Die Zugabe von Calciumionen wird in der EP 0 106 269 als unbedingt erforderlich angesehen. So offenbart die EP 0 106 269, das Ca²⁺ Fraktionen des antihämophilen Kryopräzipitats, in dem dort beschriebenen Pasteurisierungsverfahren stabilisiert. Erfindungsgemäß ist dies nicht erforderlich.

An die Behandlung mit Di- oder Trialkylphosphaten, auch in Gegenwart erhöhter Temperatur, kann sich eine chromatographische Reinigung anschließen. Dieser chromatographische Reinigungsschritt wird vorzugsweise an Anionenaustauschermaterialien wie DEAE modifizierten Ionenaustauscherharzen durchgeführt. Der pH-Wert sollte im Bereich von 6 bis 8,5 liegen.

Die auf diese Weise angereicherten oder erhaltenen pharmakologisch bedeutsamen Proteine wie Faktor VIII, Faktor IX, Fibrinogen, Gamma-Globulin u.s.w., weisen keine aktiven Viren der Art Hepatitis-A-, Parvo- wie Parvorirus B 19, Polioviren auf.

Das erfindungsgemäße Verfahren wird anhand des folgenden Beispiels zur Inaktivierung lipidfreier Viren in einer Faktor VIII Präparation näher beschrieben.

### Beispiel 1

Handelsübliches Kryopräziptat wird auf eine mit Fractogel®-DEAE-Harz gefüllte Säule aufgetragen. Das Effluat wird aufgenommen und auf Faktor VIII-Aktivität untersucht. Danach wird die Säule mit einem Puffer mit 110 mM Natriumchlorid, 10 mM Natriumcitrat x 5 H₂O, 120 mM Glycin, 1 mM Calciumchlorid x 2 H₂O, pH-Wert 6,9 bis 7,0 (mit 1 M HCl einzustellen) gewaschen. Danach wird die Säule mit einem Puffer behandelt, der die folgende Zusammensetzung aufweist: 250 mM Natriumchlorid, 20 mM Natriumcitrat x 5 H₂O, 80 mM Glycin, 16 mM Lysin, 2,5 mM Calciumchlorid x 2 H₂O bei einem pH-Wert von 6,9 bis 7,0.

Die so erhaltene Fraktion wird mit Saccharose versetzt. Danach wird die Fraktion mit Tri-(n-butyl)phosphat/Tween 0,1 % / 0,3 % 12 Stunden bei 64°C gehalten. Dann wird erneut eine Ionenaustauscherchromatographie an TSK-Fractogel®-DEAE oder EMD-Fractogel®-TMAE durchgeführt.

Die Faktor VIII aktive Fraktion wird mit einem Puffer, der 250 mM Natriumchlorid, 20 mM Natriumcitrat x 5 H₂O, 80 mM Glycin, 16 mM Lysin, 2,5 mM Calciumchlorid x 2 H₂O enthält, eluiert.

### Beispiel 2

Die Virusinaktivierung ohne Anwesenheit von Calciumionen wird durchgeführt, indem, wie in Beispiel 1 beschrieben, das handelsübliche Kryopräzipitat behandelt wird, wobei den Puffern (Waschpuffer und Elutionspuffer) vor der Wärmebehandlung keine Calciumverbindungen zugesetzt worden sind. Die Wärmebehandlung wird danach ohne Zugabe von Calciumionen durchgeführt und dann, wie in Beispiel 1 beschrieben, weiter aufgearbeitet.

## Patentansprüche

1. Verfahren zur Inaktivierung von Viren, die nicht mit einer Lipidhülle versehen sind, in Protein enthaltenden Zusammenstellungen aus Blut, Blutplasma oder ähnlichen natürlichen Quellen, durch Behandlung der Quelle mit einer wirksamen Menge von Di- oder Trialkylphosphaten und gegebenenfalls Benetzungsmitteln, gleichzeitig oder aufeinanderfolgend bei einer erhöhten Temperatur im Bereich von 60°C bis 65°C für eine Zeitdauer von fünf Stunden bis 30 Stunden.

2. Verfahren nach Anspruch 1, wobei die Menge an Di- oder Trialkylphosphat zwischen 0,001 % und 1 % beträgt.

3. Verfahren nach mindestens einem der Ansprüche 1 und/oder 2, wobei die Zeitdauer der Wärmebehandlung mindestens 10 Stunden beträgt.

4. Verfahren nach mindestens einem der Ansprüche 1 bis 3, wobei das Protein zunächst aus der natürlichen Quelle durch chromatographische oder Präzipitations-Verfahren angereichert wird.

5. Verfahren nach mindestens einem der Ansprüche 1 bis 4, wobei vor dem Behandeln mit Di- oder Trialkylphosphaten bei gleichzeitiger Behandlung bei erhöhter Temperatur, unterstützende Substanzen wie Saccharose, Sorbitol oder kurzkettige neutrale Aminosäuren zugesetzt werden.

6. Verfahren nach Anspruch 5, wobei die Konzentration an Saccharose bis zu 200 Gew.-% beträgt.

7. Verfahren nach Anspruch 5 und/oder 6, wobei die Aminosäuren Glycin, Lysin und/oder Arginin eingesetzt werden.

8. Verfahren nach mindestens einem der Ansprüche 1 bis 7, wobei die Behandlung bei erhöhter Temperatur ohne Zusatz von Calciumionen erfolgt.

9. Verfahren nach mindestens einem der Ansprüche 1 bis 8, wobei sich an die Behandlung mit Di- oder Trialkylphosphaten und gleichzeitiger Temperaturbehandlung, ein weiterer chromatographischer Reinigungsschritt anschließt.

10. Verfahren nach mindestens einem der Ansprüche 1 bis 9, wobei der pH-Wert bei der Behandlung mit Di- oder Trialkylphosphaten und erhöhter Temperatur bei 6,0 bis 8,5 liegt.

## Claims

1. A method for the inactivation of non-lipid-coated viruses in protein-containing compositions from blood, blood plasma or similar natural sources by treating said source, simultaneously or succesively, with an effective amount of dialkyl or trialkyl phosphates and optionally surfactants at an elevated temperature in the range of from 60°C to 65°C for five hours to 30 hours.

2. The method according to claim 1, wherein the amount of said dialkyl or trialkyl phosphate is between 0.001% and 1%.

3. The method according to claim 1 and/or 2, wherein the duration of the heat treatment is at least 10 hours.

4. The method according to any of claims 1 to 3, wherein the protein is first enriched from said natural source by chromatographic or precipitation methods.

5. The method according to any of claims 1 to 4, wherein auxiliary substances, such as saccharose, sorbitol or short-chain neutral amino acids, are added prior to treatment with dialkyl or trialkyl phosphates with simultaneous treatment at elevated temperatures.

6. The method according to claim 5, wherein the concentration of saccharose is up to 200% by weight.

7. The method according to claim 5 and/or 6, wherein the amino acids glycine, lysine and/or arginine are employed.

8. The method according to any of claims 1 to 7, wherein treatment at elevated temperatures is performed without addition of calcium ions.

9. The method according to any of claims 1 to 8, wherein the treatment with said dialkyl or trialkyl phosphates with simultaneous heat treatment is followed by another chromatographic purification step.

10. The method according to any of claims 1 to 9, wherein the pH value in the treatment with dialkyl or trialkyl phosphates at elevated temperatures is from 6.0 to 8.5.

## Revendications

1. Procédé d'inactivation de virus dépourvus de membrane lipidique, dans des compositions contenant une protéine, provenant du sang, du plasma sanguin ou de sources naturelles analogues, par traitement de la source avec une quantité efficace de phosphates de dialkyle ou de trialkyle et éventuellement avec des agents mouillants, simultanément ou successivement, à une température élevée comprise entre 60°C et 65°C, pendant une durée de cinq heures à 30 heures.

2. Procédé selon la revendication 1, dans lequel la quantité de phosphate de dialkyle ou de trialkyle est comprise entre 0,001 % et 1 %.

3. Procédé selon au moins l'une des revendications 1 et/ou 2, dans lequel la durée du traitement thermique est d'au moins 10 heures.

4. Procédé selon au moins l'une des revendications 1 à 3, dans lequel on concentre d'abord la protéine provenant de la source naturelle au moyen de procédés chromatographiques ou de procédés de précipitation.

5. Procédé selon au moins l'une des revendications 1 à 4, dans lequel on ajoute des substances stimulantes, comme le saccharose, le sorbitol ou des amino-acides neutres à chaîne courte, avant le traitement avec des phosphates de dialkyle ou de trialkyle et le traitement simultané à une température élevée.

6. Procédé selon la revendication 5, dans lequel la concentration de saccharose vaut jusqu'à 200 % en poids.

7. Procédé selon la revendication 5 et/ou la revendication 6, dans lequel on utilise les amino-acides glycine, lysine et/ou arginine.

8. Procédé selon au moins l'une des revendications 1 à 7, dans lequel on effectue le traitement à une température élevée sans addition d'ions calcium.

9. Procédé selon au moins l'une des revendications 1 à 8, dans lequel on effectue une étape supplémentaire de purification par chromatographie directement après le traitement avec des phosphates de dialkyle ou de trialkyle et le traitement thermique simultané.

10. Procédé selon au moins l'une des revendications 1 à 9, dans lequel la valeur de pH est de 6,0 à 8,5 pendant le traitement avec des phosphates de dialkyle ou de trialkyle et à une température élevée.
